# EUROPÄISCHE PATENTANMELDUNG

(11) **EP 1 475 043 A1**
(43) Veröffentlichungstag der Anmeldung: **10.11.2004**
(21) Anmeldenummer: 03010177.8
(22) Anmeldetag: 06.05.2003
(51) Int. Cl.: A61B 17/02

(54) **Distraktionsvorrichtung**

(71) Anmelder: Centerpulse Orthopedics Ltd., 6340 Baar (CH)
(72) Erfinder: Stutz, Heinrich, CH-8500 Frauenfeld (CH); Dominati, Simon-Paul, 13007 Marseille (FR); Gyssler, Bernhard, 8800 Thalwil (CH); Houdemer, Hervé, 8400 Winterthur (CH); Romero, José, 8700 Küsnacht (CH); Castelli, Claudio, 24129 Bergamo (IT)
(74) Vertreter: Manitz, Finsterwald & Partner GbR

(57) **Zusammenfassung**

Die Anmeldung betrifft eine hydraulische Distraktionsvorrichtung für Operationen, insbesondere für Knieoperationen, mit wenigstens einem Paar relativ zueinander verstellbarer Arbeitsabschnitte (11,13) und zumindest einer auswechselbaren Kolben-/Zylinder-Anordnung (15,17) aus Kunststoff, deren Kolben (15) mit dem einen Arbeitsabschnitt (12) und deren Zylinder (17) mit dem anderen Arbeitsabschnitt (13) lösbar gekoppelt und die durch eine äußere, den Zylinder (17) von außen abstützende Stützaufnahme (19) sowie durch wenigstens einen inneren, zusammen mit dem Kolben (15) in den Zylinder (17) einführbaren Verstärkungseinsatz (21,23,25) versteift ist.

## Beschreibung

Die Erfindung betrifft eine hydraulische Distraktionsvorrichtung für Operationen, insbesondere für Knieoperationen.

Im Rahmen von Knieoperationen einsetzbare Distraktionseinrichtungen sind grundsätzlich bekannt und dienen dazu, das Knie aufzuspreizen, d.h. den Abstand zwischen Femur und Tibia zu vergrößern. Hiermit wird bezweckt, während der Knieoperation das Femur und die Tibia in ihre natürliche Relativlage zu bringen. Der Operateur versucht dabei, diese natürliche Relativlage anhand der natürlichen Spannung der Bänder zu ermitteln. Hierfür stehen dem Operateur keine zusätzlichen Hilfsmittel zur Verfügung, d.h. der Operateur legt die der weiteren Operation zugrunde liegende Relativlage zwischen Femur und Tibia ausschließlich "nach Gefühl" fest.

Auch bei anderen am Knie durchführenden Operationen ist der Operateur mangels zur Verfügung stehender zusätzlicher Hilfsmittel auf sein "Gefühl" angewiesen. So kommt es beispielsweise bei Implantationen von vorderen Kreuzbändern darauf an, dem Band beim Einsetzen eine Bandspannung zu verleihen, die dem natürlichen Bewegungsablauf des Kniegelenks entspricht.

Aufgabe der Erfindung ist es, eine Distraktionsvorrichtung für Operationen zu schaffen, die bei einfachem Aufbau und einfacher Handhabbarkeit die Möglichkeit bietet, dem Operateur während der Operation zusätzliche Informationen zur Verfügung zu stellen.

Die Lösung dieser Aufgabe erfolgt durch die Merkmale des Anspruchs 1 und insbesondere dadurch, dass die Distraktionsvorrichtung wenigstens ein Paar relativ zueinander verstellbarer Arbeitsabschnitte und zumindest eine auswechselbare Kolben-/Zylinder-Anordnung aus Kunststoff umfasst, deren Kolben mit dem einen Arbeitsabschnitt und deren Zylinder mit dem anderen Arbeitsabschnitt lösbar gekoppelt und die durch eine äußere, den Zylinder von außen abstützende Stützaufnahme sowie durch wenigstens einen inneren, zusammen mit dem Kolben in den Zylinder einführbaren Verstärkungseinsatz versteift ist.

Die Erfindung stellt eine hydraulische Distraktionsvorrichtung bereit, die sich dadurch auszeichnet, dass die Kolben-/Zylinder-Anordnung auswechselbar und aus Kunststoff hergestellt ist. Dies ermöglicht in vorteilhafter Weise die Verwendung kostengünstiger Wegwerfteile für die Kolben-/Zylinder-Anordnung, die folglich nicht wiederholt sterilisiert zu werden brauchen.

Die erfindungsgemäß vorgesehene Stützaufnahme und der zusammen mit dem Kolben in den Zylinder einführbare Verstärkungseinsatz erhöhen die Biegesteifigkeit der Kolben-/Zylinder-Anordnung. Hierdurch ist die Distraktionsvorrichtung trotz der Herstellung der eigentlichen Kolben-/Zylinder-Anordnung aus Kunststoff aufgrund der erfindungsgemäßen Versteifung insgesamt in der Lage, vergleichsweise große Kräfte aufzunehmen, so dass während der Operation auf die mit dem Kolben und dem Zylinder verbundenen Arbeitsabschnitte einwirkende äußere Kräfte von der Stützaufnahme und dem Verstärkungseinsatz aufgenommen werden können. Nicht akzeptable Verformungen der Kolben-/Zylinder-Anordnung werden hierdurch sicher vermieden, so dass zu jedem Zeitpunkt der Operation definierte geometrische Verhältnisse herrschen und eine insbesondere vorgesehene Parallelität von zungenförmigen Plattformen, die von den Arbeitsabschnitten gebildet werden, stets gegeben ist.

Bevorzugte Ausführungsformen der Erfindung sind in den Unteransprüchen, der Beschreibung sowie der Zeichnung angegeben.

So können der Kolben und der Zylinder jeweils im Spritzgussverfahren hergestellte Einwegteile sein. Sollte die Kolben-/Zylinder-Anordnung weitere Bestandteile umfassen, so sind vorzugsweise alle diese Bestandteile für eine Einmal-Verwendung vorgesehen, wodurch kostengünstige Herstellung und - aufgrund des Wegfalls der Notwendigkeit zu wiederholter Sterilisation - einfache Handhabung optimal kombiniert werden.

Vorzugsweise sind die Stützaufnahme und der Verstärkungseinsatz aus einem Material mit einer höheren Biegesteifigkeit als der Kunststoff der Kolben-/Zylinder-Anordnung hergestellt. Insbesondere wird hierzu Metall oder faserverstärkter Kunststoff verwendet.

Der Kolben und der Verstärkungseinsatz können im zusammengesetzten Zustand eine kompakte Kolbeneinheit bilden, die zumindest über einen Teilumfangsbereich das Innere des Zylinders vollständig ausfüllt.

In einem besonders bevorzugten Ausführungsbeispiel ist die Kolben-/Zylinder-Anordnung von einer handelsüblichen Einwegspritze gebildet. Hierdurch wird auf besonders elegante Weise ein äußerst kostengünstig herstellbarer Massenartikel zu einem zentralen Bestandteil einer hydraulischen Distraktionsvorrichtung für Knieoperationen.

Ferner kann erfindungsgemäß vorgesehen sein, dass ein zwischen der Innenwand des Zylinders und einer Kolbenstange vorhandener Freiraum durch den Verstärkungseinsatz zumindest teilweise ausgefüllt ist. Hierdurch können ohnehin vorhandene Freiräume, wie sie handelsübliche Einwegspritzen aufweisen, für eine versteifende Verstärkung oder Hinterfütterung mittels des wenigstens einen Verstärkungseinsatzes genutzt werden.

Des Weiteren kann wenigstens eine im Zylinder vorhandene Kammer, die durch eine bis an die Innenwand des Zylinders reichende, insbesondere im Querschnitt kreuz- oder X-förmige Rippenstruktur einer Kolbenstange begrenzt ist, durch den Verstärkungseinsatz zumindest teilweise ausgefüllt sein.

Dabei kann wenigstens ein Paar einander gegenüberliegender Kammern jeweils durch einen Verstärkungseinsatz zumindest teilweise ausgefüllt sein.

Des Weiteren wird erfindungsgemäß vorgeschlagen, dass der Verstärkungseinsatz das Innere einer rohrförmigen, an der Innenwand des Zylinders geführten Kolbenstange zumindest über einen Teilumfangsbereich vollständig ausfüllt.

Es ist erfindungsgemäß nicht zwangsläufig erforderlich, den Kolben derart zu verstärken, dass der Zylinder über seine gesamte Querschnittsfläche vollständig ausgefüllt ist. Bevorzugt ist es, wenn sich die innere Verstärkung über die gesamte zur Verfügung stehende Länge der Kolben-/Zylinder-Anordnung erstreckt. Hierdurch ist die Stabilität und Biegesteifigkeit der Kolben-/Zylinder-Anordnung für jede Kolbenstellung und insbesondere auch bei vergleichsweise weit ausgefahrenem Kolben gewährleistet.

Die Kolben-/Zylinder-Anordnung kann derart ausgebildet sein, dass sie im zusammengesetzten Zustand mit den Arbeitsabschnitten gekoppelt werden kann. Hierdurch wird die Handhabbarkeit der erfindungsgemäßen Distraktionsvorrichtung erheblich erleichtert.

Wenigstens ein Verstärkungseinsatz kann fest mit dem Arbeitsabschnitt verbunden sein. Dies ermöglicht eine besonders stabile Konstruktion von hoher Steifigkeit.

Des Weiteren kann wenigstens ein Verstärkungseinsatz in Form eines separaten Bauteils vorgesehen sein.

Die Stützaufnahme für den Zylinder und der mit dem Zylinder koppelbare Arbeitsabschnitt können fest mit einem Tragkörper verbunden sein. Die Anzahl einzelner Bauteile der erfindungsgemäßen Distraktionsvorrichtung wird hierdurch besonders gering gehalten, was die Handhabung und insbesondere die Sterilisation der Vorrichtung in vorteilhafter Weise erleichtert.

Auf der den Arbeitsabschnitten gegenüberliegenden Seite der Kolben-/Zylinder-Anordnung kann ein Handgriff vorgesehen sein, der bevorzugt fest mit dem Tragkörper verbunden ist.

Ferner wird erfindungsgemäß vorgeschlagen, dass der mit dem Zylinder koppelbare Arbeitsabschnitt fest mit einem sich parallel zur Distraktionsrichtung erstreckenden Funktionsblock verbunden ist, der als Verdrehund/oder Auslenksicherung für den mit dem Kolben koppelbaren Arbeitsabschnitt ausgebildet ist.

Durch einen derartigen Funktionsblock kann für alle Kolbenstellungen eine korrekte Relativlage zwischen den beiden Arbeitsabschnitten auch bei relativ großen auf die Arbeitsabschnitte einwirkenden äußeren Kräften sichergestellt werden.

Der Funktionsblock kann einen von einer Kreisform abweichenden Außenquerschnitt aufweisen und zumindest über einen Teilumfangsbereich von einem Führungsabschnitt des Arbeitsabschnitts verdrehsichernd umgriffen sein.

Ferner kann vorgesehen sein, dass der Funktionsblock eine geringfügig gegenüber der Distraktionsrichtung geneigte, in Richtung zunehmender Distraktionslänge auf die Längsachse der Kolben-/Zylinder-Anordnung zu laufende Anschlagfläche aufweist, die mit dem Arbeitsabschnitt auslenkungssichernd zusammenwirkt.

Vorzugsweise ist der Funktionsblock mit einer Anzeigeeinrichtung versehen, an der ein Maß für die Distraktionslänge ablesbar ist. Die Anzeigeeinrichtung kann in Form einer an einer Außenseite des Funktionsblocks angebrachten Skala vorgesehen sein. Indem auf diese Weise ein Maß für die Distraktionslänge und damit für den Abstand zwischen den distrahierten Arbeitsabschnitten zur Verfügung steht, können Knieoperationen auf einfache und zuverlässige Weise reproduzierbar gestaltet werden.

Des Weiteren kann die Kolben-/Zylinder-Anordnung mit einer Anzeigeeinrichtung, insbesondere in Form einer am Zylinder angebrachten Skala, versehen sein. Im Fall der Verwendung einer Einwegspritze als Kolben-/ Zylinder-Anordnung kann die ohnehin am Zylinder der Spritze angebrachte Skala genutzt werden.

Des Weiteren wird erfindungsgemäß vorgeschlagen, dass eine Fluidzuführeinrichtung für die Kolben-/Zylinder-Anordnung eine von Hand betätigbare Spritze umfasst, insbesondere eine Einwegspritze.

An eine die Kolben-/Zylinder-Anordnung mit einer Fluidzuführeinrichtung verbindende Fluidleitung kann wenigstens eine Druckanzeigeeinrichtung angeschlossen sein. Hierdurch steht ein Maß für die zwischen den Arbeitsabschnitten wirksame Distraktionskraft zur Verfügung. Werden bei der Operation Bänder gespannt, so kann folglich an der Druckanzeigeeinrichtung ein Maß für die jeweilige Bandspannung abgelesen werden. Dies ermöglicht es wiederum, die Operationen reproduzierbar zu gestalten.

In einer weiteren bevorzugten Ausführungsform der Erfindung ist die Kolben-/Zylinder-Anordnung ein Bestandteil eines geschlossenen, als eine Einheit handhabbaren Hydrauliksystems, das zusätzlich zumindest eine Fluidzuführeinrichtung, eine die Kolben-/Zylinder-Anordnung mit der Fluidzuführeinrichtung verbindende Fluidleitung sowie ein Hydraulikfluid, insbesondere Wasser, umfasst und als Ganzes mit den Arbeitsabschnitten koppelbar ist. Ein solches geschlossenes Hydrauliksystem kann insbesondere als Ganzes sterilisiert und mit den übrigen Bauteilen oder Baugruppen der Distraktionsvorrichtung gekoppelt werden, was die Benutzung erheblich erleichtert. Bei der Vorbereitung oder während der Operation braucht das vorher sterilisierte Hydrauliksystem lediglich der Verpackung entnommen und mit den Arbeitsabschnitten gekoppelt zu werden. Umständliche und zeitraubende Anschluss- oder Entlüftungsarbeiten sind nicht erforderlich.

Gemäß einem weiteren Ausführungsbeispiel der Erfindung sind wenigstens zwei im mit den Arbeitsabschnitten gekoppelten Zustand in parallelen Richtungen wirksame Kolben-/Zylinder-Anordnungen vorgesehen.

Eine Vorrichtung mit zwei nebeneinander liegenden Kolben-/Zylinder-Anordnungen jeweils mit zugehörigen Arbeitsabschnitten kann insbesondere für bikompartimentale Operationen zum Einsetzen von bikondylären Knieprothesen verwendet werden.

Bevorzugt sind die Kolben-/Zylinder-Anordnungen unabhängig voneinander ansteuerbar, d.h. mit Hydraulikfluid beaufschlagbar, so dass der Abstand zwischen Femur und Tibia in den beiden Kompartimenten unterschiedlich groß eingestellt werden kann.

Bevorzugt sind die Kolben-/Zylinder-Anordnungen während der Benutzung an eine gemeinsame Fluidzuführeinrichtung angeschlossen.

Ferner können durch in einer die Kolben-/Zylinder-Anordnungen mit einer Fluidzuführeinrichtung verbindenden Fluidleitung angeordnete Schaltventile die Kolben-/Zylinder-Anordnungen unabhängig voneinander jeweils wahlweise mit der Fluidzufuhr verbindbar oder von der Fluidzufuhr trennbar sein.

Zum Einstellen von Sollabständen zwischen Femur und Tibia sind die Arbeitsabschnitte bevorzugt derart ausgebildet, dass sie einen zwischen Femur und Tibia einbringbaren und sich im Wesentlichen senkrecht zur Distraktionsrichtung erstreckenden Aufspreizabschnitt bilden. Die Arbeitsabschnitte können jeweils in Form einer plattenförmigen Zunge vorgesehen sein.

Die erfindungsgemäße Distraktionsvorrichtung ist nicht ausschließlich zum Auseinanderdrücken von Tibia und Femur verwendbar, sondern kann auch im Rahmen anderer Operationen insbesondere im Bereich des Knies verwendet werden, bei denen Teile relativ zueinander bewegt werden sollen und es wünschenswert ist, während des Operationsverlaufs zusätzliche Informationen insbesondere über die momentanen Werte der Distraktionslänge der Distraktonskraft zu erhalten.

So kann die erfindungsgemäße Distraktionsvorrichtung beispielsweise bei der Implantation vorderer Kreuzbänder dazu verwendet werden, das am Femur fixierte Band zu spannen, um die Fixierung des Bandes an der Tibia bei einer für die natürliche Bewegung des Kniegelenks passenden Bandspannung durchzuführen.

Zur Bestimmung der Bandspannung und/oder Bandlänge bei der Implantation von vorderen Kreuzbändern kann ein oberer Arbeitsabschnitt mit einem Abstütz- und/oder Befestigungsabschnitt versehen sein, über den die Vorrichtung an der Tibia abstützbar und/oder befestigbar ist.

Die Arbeitsabschnitte können jeweils mit wenigstens einer Aussparung zum Hindurchführen eines Fixierwerkzeugs und eines Fixierelementes zum Fixieren des Bandes in der Tibia versehen sein. Bei dem Werkzeug handelt es sich beispielsweise um einen Schraubendreher, mit dem eine das Fixierelement bildende Verdrängerschraube in eine in der Tibia ausgebildete Bohrung eingeschraubt werden kann, um das zu implantierende, in die Bohrung eingeführte Band festzusetzen. Die in den Arbeitsabschnitten ausgebildete Aussparung ist vorzugsweise derart bemessen, dass der Schraubendreher mit aufgesetzter Verdrängerschraube entlang dem Band eingebracht werden kann.

Des Weiteren wird vorgeschlagen, dass der obere Arbeitsabschnitt mit wenigstens einer Aussparung zum Hindurchführen des Bandes und/oder eines mit dem Band verbundenen Zugelementes versehen ist.

Ferner kann ein unterer Arbeitsabschnitt mit wenigstens einem Befestigungsabschnitt zum Befestigen des Bandes oder eines mit dem Band verbundenen Zugelementes versehen sein.

Die Erfindung wird im Folgenden beispielhaft unter Bezugnahme auf die Zeichnung beschrieben. Es zeigen:
- Fig. 1: einen Längsschnitt durch eine Distraktionsvorrichtung gemäß einer Ausführungsform der Erfindung,
- Fig. 2: eine Vorderansicht der Distraktionsvorrichtung von Fig. 1,
- Fig. 3: eine Draufsicht auf die Distraktionsvorrichtung von Fig. 1 ohne Zylinder und ohne obere Arbeitsabschnitte,
- Fig. 4: eine Draufsicht entsprechend Fig. 3 mit eingeführten Zylindern und oberen Arbeitsabschnitten,
- Fig. 5: eine Möglichkeit für den Anschluss einer erfindungsgemäßen Distraktionsvorrichtung an eine Fluidzuführeinrichtung,
- Fig. 6: eine Möglichkeit für den Anschluss einer erfindungsgemäßen Distraktionsvorrichtung an zwei unabhängig voneinander betreibbare Fluidzuführeinrichtungen,
- Fig. 7: einen Teillängsschnitt durch eine Distraktionsvorrichtung gemäß einer weiteren Ausführungsform der Erfindung,
- Fig. 8: die Distraktionsvorrichtung von Fig. 7 als Bestandteil eines geschlossenen Hydrauliksystems, und
- Fig. 9 - 11: die Verwendung erfindungsgemäßer Distraktionsvorrichtungen bei der Implantation eines vorderen Kreuzbandes.

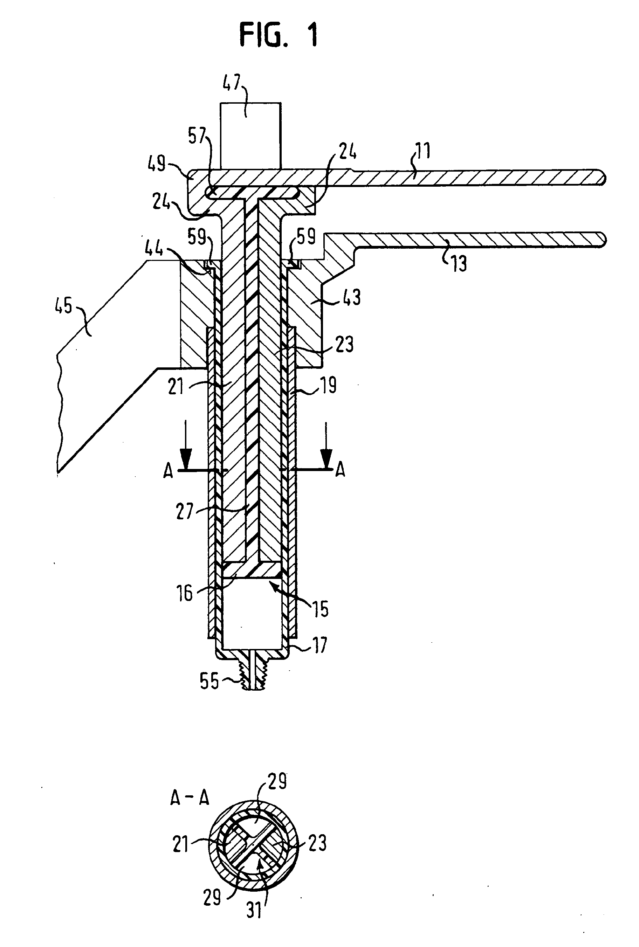

Die in den Fig. 1 - 4 dargestellte Ausführungsform einer erfindungsgemäßen hydraulischen Distraktionsvorrichtung umfasst einen Tragkörper 43, der auf einer Seite einen Handgriff 45 trägt und auf der gegenüberliegenden Seite einteilig mit einer zungenförmigen Plattform 13 verbunden ist, die einen unteren Arbeitsabschnitt der Distraktionsvorrichtung bildet.

Im Tragkörper 43 sind ferner zwei parallel verlaufende Durchgänge ausgebildet, die jeweils nach unten in ein am Tragkörper 43 befestigtes Stützrohr 19 übergehen. Auf der von den Stützrohren 19 abgewandten Seite weist der Tragkörper 43 einen mittig zwischen den Durchgängen angeordneten Funktionsblock 47 auf, auf den an anderer Stelle näher eingegangen wird.

In die Durchgänge des Tragkörpers 43 eingeführt ist jeweils ein Zylinder 17 einer in diesem Ausführungsbeispiel in Form einer handelsüblichen Einwegspritze vorgesehenen Kolben-/Zylinder-Anordnung. In ihrer Sollstellung stützen sich die Zylinder 17 jeweils mit Laschen 59 an einer entsprechend ausgebildeten Ringschulter 44 des Tragkörpers 43 ab. Die Zylinder 17 liegen jeweils mit ihrer Außenwand an der Innenwand des Stützrohres 19 an. Auf jeweils am freien Ende der Zylinder 17 ausgebildete Anschlussabschnitte 55 sind in den Fig. 1 - 4 nicht dargestellte Fluidleitungen aufschraubbar.

Jede Einwegspritze umfasst des Weiteren einen Kolben 15 mit einem an der Innenwand des Zylinders 17 fluiddicht anliegenden Kolbenabschnitt 16 und einer Kolbenstange 27, die an ihrem vom Kolbenabschnitt 16 abgewandten freien Ende in einen auch als Pilz bezeichneten, radial gegenüber der Kolbenstange 27 erweiterten Kopfabschnitt 57 übergeht.

Die Führung des Kolbens 15 im Bereich der Kolbenstange 27 erfolgt durch eine kreuzförmige Rippenstruktur 31 (vgl. die Querschnittsansicht A-A unten in Fig. 1). Die Kolbenstange 27 umfasst vier Rippen, wobei jeweils zwei benachbarte Rippen einen Winkel von 90° einschließen. Bei in den Zylinder 17 eingeschobenem Kolben 15 begrenzen die Rippen der Kolbenstange 27 Kammern 29 innerhalb des Zylinders 17. In dem dargestellten Ausführungsbeispiel sind zwei einander gegenüberliegende Kammern (vgl. die Querschnittsansicht A-A) jeweils vollständig durch einen Verstärkungseinsatz 21 bzw. 23 ausgefüllt.

Der eine Verstärkungseinsatz 21 ist einstückig mit einer weiteren zungenförmigen Plattform 11 verbunden, die den oberen Arbeitsabschnitt der erfindungsgemäßen Distraktionsvorrichtung bildet. Der andere Verstärkungseinsatz 23 ist in Form eines separaten Bauteils vorgesehen. Der mit der oberen Plattform 11 verbundene Einsatz 21, der separate Einsatz 23 sowie der Kolben 15 werden zunächst zu einer Kolbeneinheit zusammengesetzt, bevor diese als Ganzes in den Zylinder 17 eingeschoben wird, wobei der Zylinder 17 zuvor bereits in den entsprechenden Durchgang des Tragkörpers 43 eingeschoben worden sein kann.

Beim Zusammensetzen der Kolbeneinheit wird der pilzförmige Kopf 57 der Kolbenstange 27 zunächst mit einer Seite in eine schlitzförmige Aufnahme zwischen der oberen Plattform 11 und einem sich parallel zur oberen Plattform 11 erstreckenden Flanschabschnitt 24 eingeführt, der Bestandteil eines Übergangsabschnitts 49 zwischen der oberen Plattform 11 und dem mit dieser verbundenen Verstärkungseinsatz 21 ist.

Der separate Verstärkungseinsatz 23 ist mit einem entsprechenden Flanschabschnitt 24 versehen, so dass der Kopf 57 der Kolbenstange 27 zwischen den beiden Einsätzen 21, 23 und der oberen Plattform 11 räumlich fixiert ist.

Beim Einführen des Kopfes 57 in den im Übergangsabschnitt 49 ausgebildeten Aufnahmeschlitz gelangt der mit der oberen Plattform 11 verbundene Einsatz 21 in die entsprechende Kammer (vgl. Querschnittsansicht A-A in Fig. 1) der Rippenstruktur 31. Entsprechendes gilt beim Einfügen des separaten Einsatzes 23.

Mit den beiden Einsätzen 21, 23 werden folglich für jede Stellung des Kolbens 15 zwei einander gegenüberliegende Kammern innerhalb des Zylinders 17 vollständig mit Material ausgefüllt, wodurch der Kolbeneinheit eine hohe Biegesteifigkeit verliehen wird. Durch das Stützrohr 19 wird der Zylinder 17 ferner von außen abgestützt, so dass insgesamt eine biegesteife Kolben-/Zylinder-Anordnung geschaffen wird, die auch bei großen auf die beiden Plattformen 11, 13 einwirkenden äußeren Kräften stets eine ausreichende Parallelität zwischen den beiden Plattformen 11, 13 sicherstellt. Dabei dient in vorteilhafter Weise das obere freie Ende des separaten Einsatzes 23 im Bereich des Flanschabschnitts 24 als Abstützung für die obere Plattform 11.

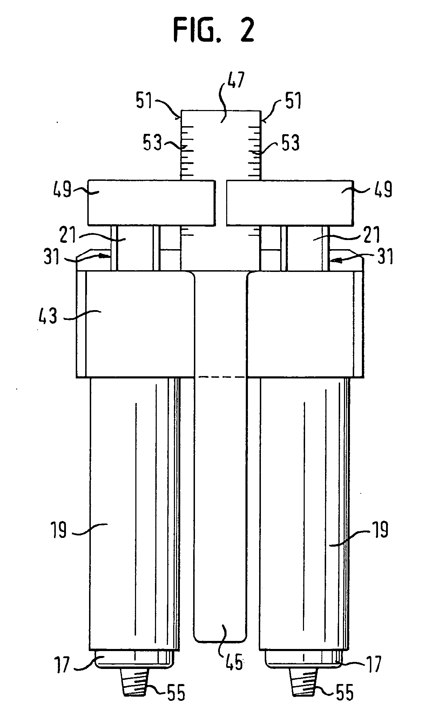

Die Vorderansicht der Fig. 2 zeigt insbesondere den mittig bezüglich der Stützrohre 19, aus denen die Zylinder 17 unten jeweils ein Stück weit heraus ragen, angeordneten Handgriff 45. Außerdem in Fig. 2 zu erkennen sind die in Richtung des Handgriffs 45 weisenden Seiten der fest mit der oberen Plattform 11 verbundenen Einsätze 21 sowie jeweils der beiden die Kammer für den Einsatz 21 begrenzenden Rippen der Rippenstruktur 31.

Der Funktionsblock 47 ist auf seiner in Richtung des Handgriffs 45 weisenden Seite mit Skalen 53 versehen, an denen die jeweilige Kolbenstellung abgelesen werden kann, wodurch ein Maß für die Distraktionslänge zur Verfügung steht.

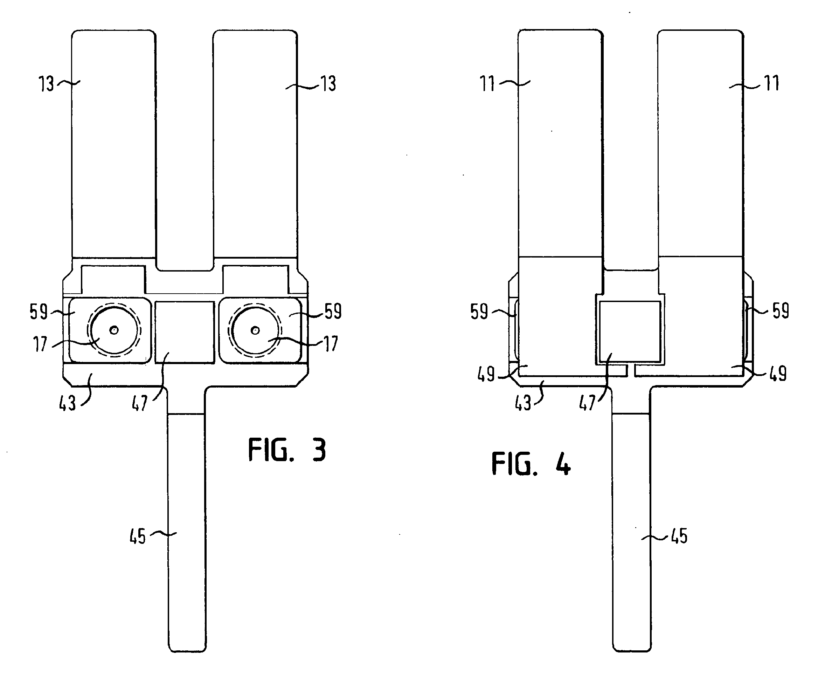

Wie insbesondere aus Fig. 4 hervorgeht, wird der Funktionsblock 47 von den Übergangsabschnitten 49 jeweils in einen Eckbereich umgriffen und dient somit als eine Führung für die jeweils aus Kolbeneinheit und oberer Plattform 11 bestehenden Baugruppen. Insofern stellen die Übergangsabschnitte 49 jeweils einen Führungsabschnitt für die betreffende Baugruppe dar, der ein Verdrehen der Baugruppe relativ zu dem Tragkörper 43 um die Längsachse der Kolben-/Zylinder-Einheit sicher verhindert und somit eine gleichbleibende Orientierung der jeweiligen oberen Plattform 11 sicherstellt.

Abweichend von der dargestellten Ausführungsform kann der Funktionsblock 47 derart trapezförmig ausgebildet sein, dass er sich nach oben verbreitert, so dass - anders als in Fig. 2 - die Außenseiten 53 nicht parallel, sondern geneigt zur Distraktionsrichtung verlaufen. Ein bei vollständig eingeschobener Kolbeneinheit noch vorhandenes, geringes Spiel zwischen Führungsabschnitt 49 und Funktionsblock 47 wird auf diese Weise mit zunehmender Distraktionslänge reduziert.

Fig. 3, in der die erfindungsgemäße Distraktionsvorrichtung ohne in die Zylinder 17 eingeschobene Kolbeneinheiten und damit ohne obere Plattformen 11 dargestellt ist, zeigt insbesondere die bezüglich der Längsachse rechtwinklig abstehenden Laschen 59, mit denen die Zylinder 17 jeweils auf den hierfür vorgesehenen Ringschultern 44 des Tragkörpers 43 aufliegen.

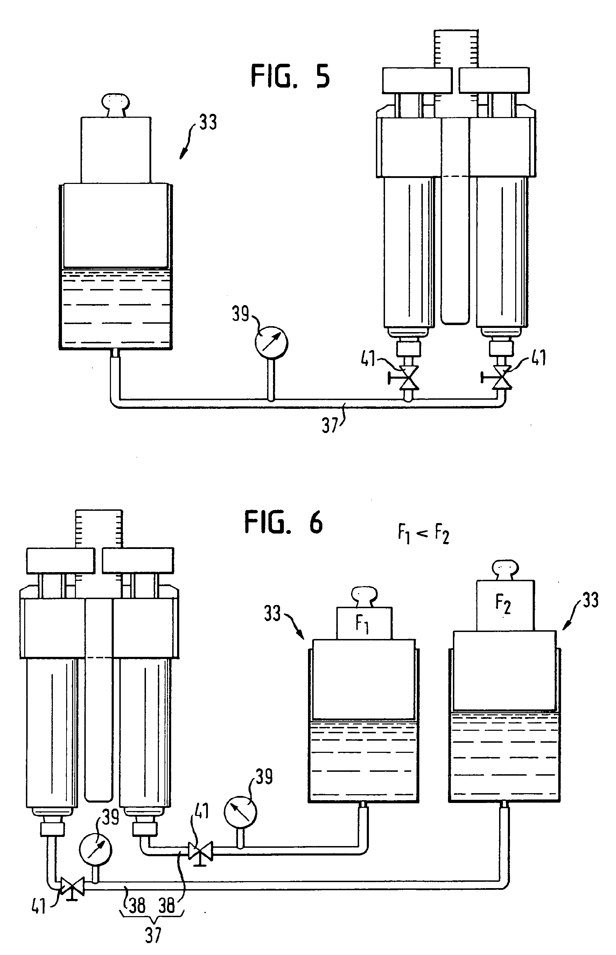

Wie Fig. 5 zeigt, können beide Kolben-/Zylinder-Anordnungen an eine gemeinsame, hier nur prinzipiell dargestellte Fluidzuführeinrichtung 33 angeschlossen werden. Schaltventile 41 ermöglichen es, die beiden Einheiten insofern unabhängig voneinander zu betreiben, als die eine Einheit durch Schließen des betreffenden Ventils 41 bei der gerade erreichten Distraktionslänge festgesetzt und bei der anderen Einheit durch Offenlassen des Ventils 41 die Distraktion fortgesetzt wird. Hierdurch kann beispielsweise beim Aufspreizen eines Knies mit einer asymmetrischen Kräfteverteilung gearbeitet werden.

An einer an die gemeinsame Fluidleitung 37 angeschlossenen Druckanzeigeeinrichtung 39 kann ein Maß für die zwischen den Arbeitsabschnitten wirksame Distraktionskraft abgelesen werden.

Fig. 6 zeigt ein Beispiel, bei dem die beiden Kolben-/Zylinder-Anordnungen der Distraktionsvorrichtung vollkommen unabhängig voneinander betrieben werden können. Jeder Einheit ist eine eigene Fluidzuführeinrichtung 33 zugeordnet, die über eine Einzelleitung 38 an den jeweiligen Zylinder angeschlossen ist. Jeder Einzelleitung 38 ist ein Schaltventil 41 zum wahlweisen Absperren oder Freigeben der Einzelleitung 38 sowie eine Druckanzeigeeinrichtung 39 zugeordnet.

Bei den in Fig. 5 und 6 dargestellten Fluidzuführeinrichtungen 33 kann es sich um handelsübliche Einwegspritzen 33 handeln. Diese können von gleicher Bauart sein wie die die Kolben-/Zylinder-Anordnungen 15, 17 bildenden Einwegspritzen, sofern das insgesamt für die beiden Kolben-/Zylinder-Anordnungen 15, 17 benötigte Fluidvolumen mit einer einzigen Einwegspritze zur Verfügung gestellt werden kann. Zumindest für den überwiegenden Teil von unter Zuhilfenahme der erfindungsgemäßen Distraktionsvorrichtung durchführbaren Operationen ist dies der Fall, da man in den meisten Fällen ohnehin bestrebt ist, ein übermäßiges Ausfahren der Kolbeneinheiten zu vermeiden.

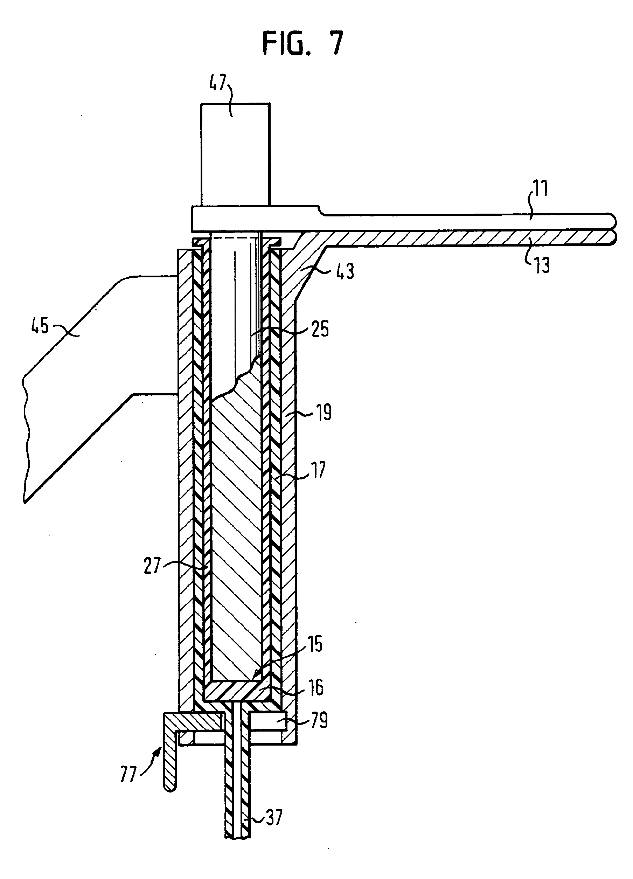

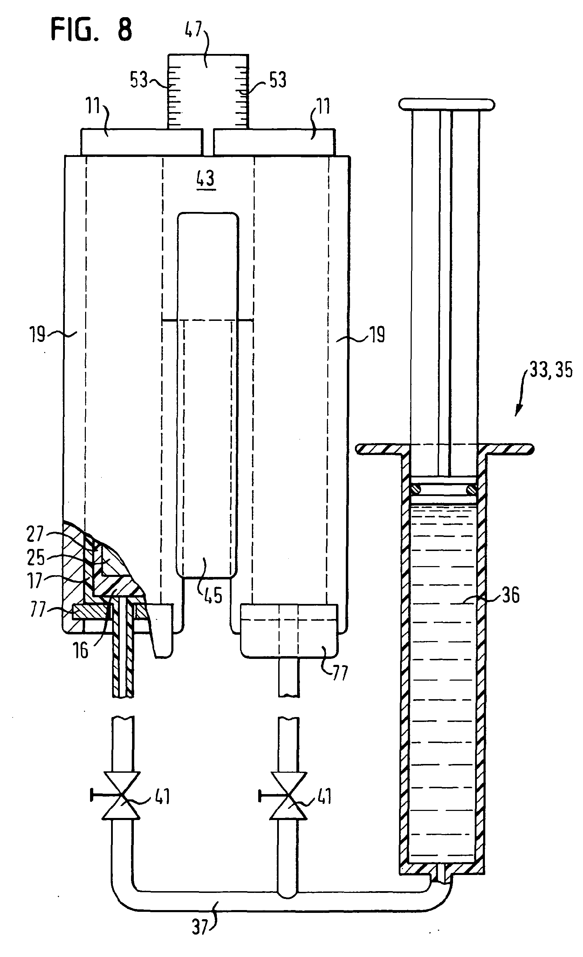

In dem weiteren Ausführungsbeispiel einer erfindungsgemäßen Distraktionsvorrichtung gemäß Fig. 7 und 8 ist lediglich ein einziger Verstärkungseinsatz 25 vorgesehen, der fest und insbesondere einteilig mit der oberen Plattform 11 ausgebildet ist. Der Einsatz 25 ist ein Vollzylinder, der mit seiner Außenwand an der Innenwand einer in diesem Ausführungsbeispiel rohrförmigen Kolbenstange 27 anliegt, so dass das Innere der Kolbenstange 27 durch den Einsatz 25 vollständig ausgefüllt ist.

Der einteilig mit einer in Fig. 7 nur teilweise dargestellten Fluidleitung 37 verbundene Zylinder 17 der Kolben-/Zylinder-Anordnung wird in dieser Ausführungsform von unten in das mit dem Tragkörper 43 verbundene Stützrohr 19 eingeführt. Eine Verriegelungseinrichtung 77 mit einem gabelförmigen Verriegelungsabschnitt 79 dient zur Sicherung des eingeschobenen Zylinders 17. Aus nachstehend erläuterten Gründen erfolgt das Einschieben des Zylinders 17 in das Stützrohr 19 bei in den Zylinder 17 eingeführtem Kolben 15, so dass zur Komplettierung der Distraktionsvorrichtung anschließend nur noch die vollzylindrischen Einsätze 25 in die rohrförmigen Kolbenstangen 27 eingeschoben zu werden brauchen.

Wie Fig. 8 zeigt, sind die beiden Kolben-/Zylinder-Anordnungen Bestandteile eines geschlossenen Hydrauliksystems, das außerdem eine gemeinsame Fluidzuführeinrichtung 33 in Form einer Einwegspritze 35, ein Hydraulikfluid 36, insbesondere Wasser, sowie eine die beiden Zylinder mit der Spritze 33 verbindende Fluidleitung 37 mit Schaltventilen 41 jeweils in Form eines Absperrhahns umfasst. Zusätzlich kann die Fluidleitung 37 mit einer oder mehreren Druckanzeigevorrichtungen versehen sein. Dieses Hydrauliksystem wird als fertig zusammengesetzte und insbesondere bereits mit dem Hydraulikfluid 36 gefüllte Einheit hergestellt und als Ganzes sterilisiert geliefert.

Die Handhabung der erfindungsgemäßen Distraktionsvorrichtung ist hierdurch für den Operateur äußerst einfach. Gegebenenfalls im Anschluss an eine Sterilisation des Nicht-Hydrauliksystems, also der übrigen, nicht zu dem vorstehend erwähnten Hydrauliksystem gehörenden Bestandteile der Distraktionsvorrichtung, brauchen lediglich die beiden Kolben-/Zylinder-Anordnungen des sterilen Hydrauliksystems von unten in die Stützrohre 19 eingeschoben und mittels der Verriegelungseinrichtungen 77 gesichert sowie die Verstärkungseinsätze 25 mit den oberen Zungen 11 von oben eingesetzt zu werden.

Abweichend von den beiden vorstehend beschriebenen Ausführungsformen kann die erfindungsgemäße Distraktionsvorrichtung auch lediglich eine einzige Kolben-/Zylinder-Anordnung 15, 17 mit einem einzigen Paar zungenförmigen Plattformen 11, 13 umfassen.

Bei den Bestandteilen der Kolben-/Zylinder-Anordnungen handelt es sich bevorzugt um im Spritzgussverfahren hergestellte Kunststoffteile, wohingegen zumindest die Stützrohre 19 und die Verstärkungseinsätze 21, 23, 25 aus einem Material höherer Biegesteifigkeit, beispielsweise aus Metall oder einem faserverstärkten Kunststoff, hergestellt sind, welche sich wegen ihrer Wiederverwendung im Spital zum Beispiel dampfsterilisieren lassen.

Die in den Fig. 1 bis 8 beschriebenen Distraktionsvorrichtungn dienen zum Aufspreizen des Knies, wie es im Einleitungsteil erläutert wurde, und ermöglichen das so genannte "Auspendeln" des Kniegelenks zur Bestimmung des durch die natürliche Spannung der Bänder bestimmten optimalen Abstandes zwischen Femur und Tibia unter gleichzeitiger Anzeige von für das betreffende Knie charakteristischen Messwerten für die momentanen Parameter Distraktionslänge und Distraktionskraft.

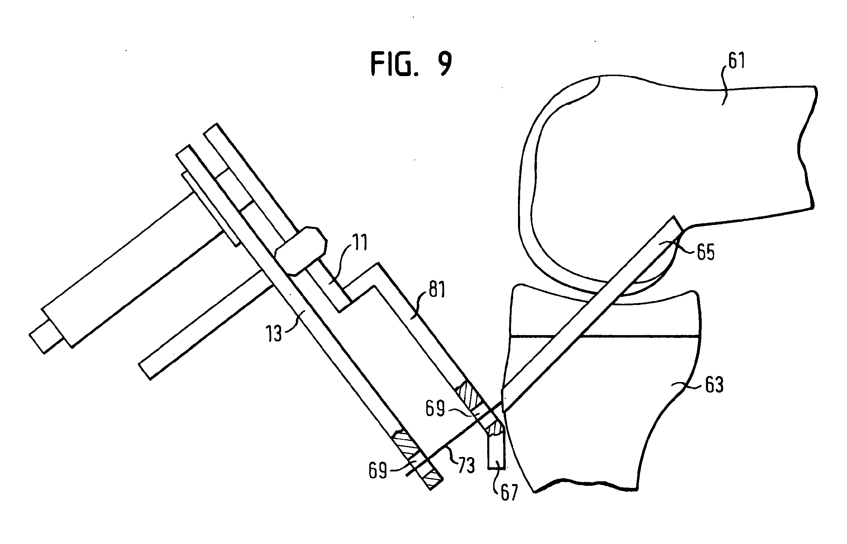

Die Fig. 9 bis 11 zeigen die Verwendung erfindungsgemäßer Distraktionsvorrichtungen beim Implantieren eines vorderen Kreuzbandes. Bei einer derartigen Operation wird das Band 65 in eine zuvor im Femur 61 ausgebildete Bohrung eingeschoben und dort mittels einer entlang der Mantellinie des Bandes 65 eingedrehten, nicht dargestellten Verdrängerschraube festgesetzt. Anschließend wird das Band durch eine zuvor in der Tibia 63 ausgebildete Bohrung nach vorne durchgezogen, um in dieser Tibiabohrung mittels einer weiteren, nicht dargestellten Verdrängerschraube festgesetzt zu werden.

Hier setzt jetzt die weitere Verwendung der erfindungsgemäßen Distraktionsvorrichtung ein:

Während es bislang üblich war, vor dem Festziehen der Tibia-Verdrängerschraube dem Band 65 eine vorgegebene Vorspannung unter Zuhilfenahme einer Federwaage zu verleihen, um das Band 65 unter dieser Vorspannung festzusetzen, wobei die Federwaage ohne weitere Abstützung nach außen gezogen wurde, wird nunmehr die Federwaage durch die erfindungsgemäße Distraktionsvorrichtung ersetzt, die sowohl eine einzige als auch - wie bei den vorstehend in Verbindung mit den Fig. 1 - 8 beschriebenen Ausführungsformen - zwei parallel angeordnete Kolben-/Zylinder-Anordnungen und damit obere Plattformen 11 aufweisen kann.

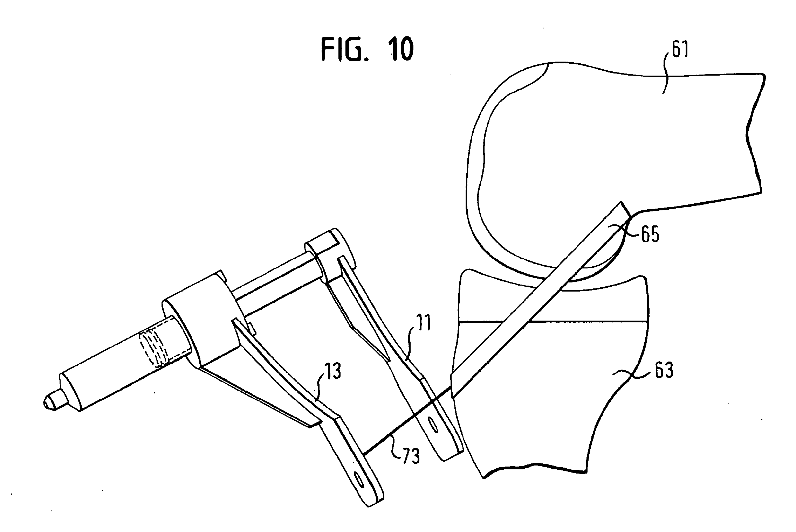

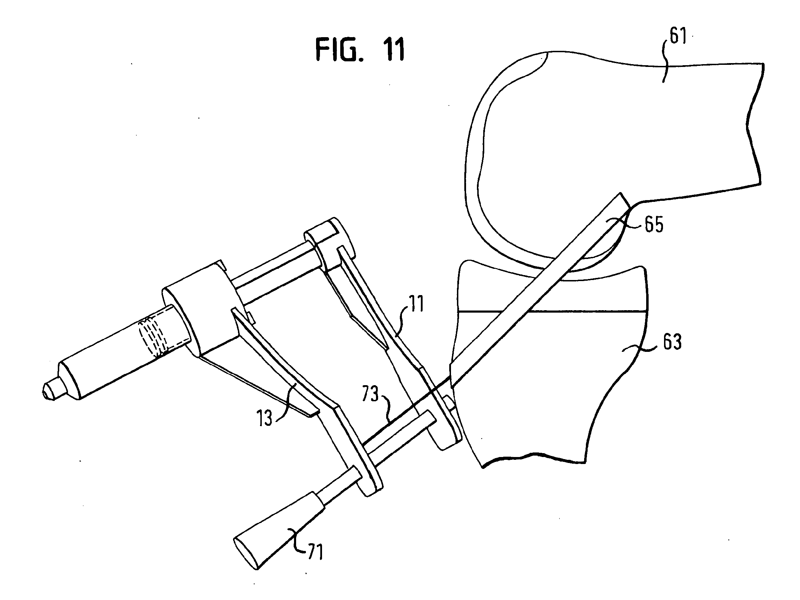

Im Folgenden wird der Einfachheit halber von einer "Mono-Variante" mit lediglich einer einzigen Kolben-/Zylinder-Anordnung ausgegangen, wie sie beispielsweise in den Fig. 10 und 11 dargestellt ist.

Während der Operation wird die Distraktionsvorrichtung mit ihrer oberen Arbeitsplattform 11, die - wie in Fig. 9 gezeigt - hierzu mit einem speziellen Aufsatz 81, dessen vorderes freies Ende von einem abgewinkelten oder abwinkelbaren Abstütz- und/oder Befestigungsabschnitt gebildet ist, versehen sein kann, an der Tibia 63 abgestützt. Das Band 65 und ggf. - wie in den Fig. 9-11 gezeigt - ein mit dem Band 65 verbundenes Zugelement 73 beispielsweise in Form eines Drahtes wird durch in beiden Plattformen 11, 13 ausgebildete Aussparungen 69 hindurch gezogen und an der unteren Plattform 13 befestigt. Die Aussparungen 69 sind dabei derart bemessen, dass ein in Fig. 11 schematisch dargestellter Schraubendreher 71 mit aufgesetzter Verdrängerschraube entlang dem Band 65 eingebracht werden kann, um das die richtige Vorspannung aufweisende Band 65 in der Tibia 63 festzusetzen.

Mittels der erfindungsgemäßen Distraktionsvorrichtung ist es in vorteilhafter Weise möglich, vor dem endgültigen Festsetzen des Bandes 65 in der Tibia 63 entweder bei allen möglichen oder zumindest bei einigen ausgewählten Stellungen, d.h. Beugungswinkeln, des Knies die Bandspannung zu messen bzw. ein Maß für die Bandspannung zu ermitteln, um ggf. durch Variieren der Bandlänge eine Bandspannung einzustellen, die für alle möglichen Beugungswinkel und damit für den normalen Bewegungsablauf des Kniegelenks geeignet ist. Das Maß für die Bandspannung wird mit einer Druckanzeigeeinrichtung ermittelt, wie sie beispielsweise in Verbindung mit den Ausführungsformen der Fig. 1 - 8 beschrieben wurde, während das Maß für die Bandlänge durch die Distraktionslänge gegeben ist, die an einer Anzeigeeinrichtung, z.B. in Form einer Skala, abgelesen werden kann, wie sie ebenfalls beispielsweise in Verbindung mit den Ausführungsformen der Fig. 1 - 8 beschrieben wurde.

Während der Ermittlung der optimalen Länge des zu implantierenden Bandes 65 kann die obere Plattform 11 beispielsweise mit Hilfe von Knochennägeln an der Tibia 63 befestigt werden, um für definierte geometrische Verhältnisse zu sorgen.

### Bezugszeichenliste

- 11: oberer Arbeitsabschnitt, obere Zunge
- 13: unterer Arbeitsabschnitt, untere Zunge
- 15: Kolben
- 16: Kolbenabschnitt
- 17: Zylinder
- 19: Stützaufnahme, Stützrohr
- 21: Verstärkungseinsatz
- 23: Verstärkungseinsatz
- 24: Flanschabschnitt
- 25: Verstärkungseinsatz
- 27: Kolbenstange
- 29: Kammer
- 31: Rippenstruktur
- 33: Fluidzuführeinrichtung
- 35: Spritze
- 36: Hydraulikfluid
- 37: Fluidleitung
- 39: Druckanzeigeeinrichtung
- 41: Schaltventil
- 43: Tragkörper
- 44: Ringschulter
- 45: Handgriff
- 47: Funktionsblock
- 49: Führungsabschnitt
- 51: Anschlagfläche
- 53: Anzeigeeinrichtung, Skala
- 55: Anschlussabschnitt
- 57: Kopfabschnitt, Pilz
- 59: Lasche
- 61: Femur
- 63: Tibia
- 65: Kreuzband
- 67: Abstütz- und/oder Befestigungsabschnitt
- 69: Aussparung
- 71: Fixierwerkzeug, Schraubendreher
- 73: Zugelement
- 77: Verriegelungseinrichtung
- 79: gabelförmiger Verriegelungsabschnitt
- 81: Aufsatz

## Patentansprüche

1. Hydraulische Distraktionsvorrichtung für Operationen, insbesondere für Knieoperationen, mit wenigstens einem Paar relativ zueinander verstellbarer Arbeitsabschnitte (11, 13) und zumindest einer auswechselbaren Kolben-/Zylinder-Anordnung (15, 17) aus Kunststoff, deren Kolben (15) mit dem einen Arbeitsabschnitt (11) und deren Zylinder (17) mit dem anderen Arbeitsabschnitt (13) lösbar gekoppelt und die durch eine äußere, den Zylinder (17) von außen abstützende Stützaufnahme (19) sowie durch wenigstens einen inneren, zusammen mit dem Kolben (15) in den Zylinder (17) einführbaren Verstärkungseinsatz (21, 23, 25) versteift ist.

2. Distraktionsvorrichtung nach Anspruch 1,
**dadurch gekennzeichnet,**
**dass** der Kolben (15) und der Zylinder jeweils im Spritzgussverfahren hergestellte Einwegartikel sind.

3. Distraktionsvorrichtung nach Anspruch 1 oder 2,
**dadurch gekennzeichnet,**
**dass** der Kolben (15) und der Verstärkungseinsatz (21, 23, 25) im zusammengesetzten Zustand eine kompakte Kolbeneinheit bilden, die zumindest über einen Teilumfangsbereich das Innere des Zylinders (17) vollständig ausfüllt.

4. Distraktionsvorrichtung nach einem der vorhergehenden Ansprüche,
**dadurch gekennzeichnet,**
**dass** die Kolben-/Zylinder-Anordnung (15, 17) von einer handelsüblichen Einwegspritze gebildet ist.

5. Distraktionsvorrichtung nach einem der vorhergehenden Ansprüche,
**dadurch gekennzeichnet,**
**dass** ein zwischen der Innenwand des Zylinders (17) und einer Kolbenstange (27) vorhandener Freiraum durch den Verstärkungseinsatz (21, 23) zumindest teilweise ausgefüllt ist.

6. Distraktionsvorrichtung nach einem der vorhergehenden Ansprüche,
**dadurch gekennzeichnet,**
**dass** wenigstens eine im Zylinder (17) vorhandene Kammer (29), die durch eine bis an die Innenwand des Zylinders (17) reichende, insbesondere im Querschnitt kreuz- oder X-förmige Rippenstruktur (31) einer Kolbenstange (27) begrenzt ist, durch den Verstärkungseinsatz (21, 23) zumindest teilweise ausgefüllt ist.

7. Distraktionsvorrichtung nach Anspruch 6,
**dadurch gekennzeichnet,**
**dass** wenigstens ein Paar einander gegenüberliegender Kammern jeweils durch einen Verstärkungseinsatz (21, 23) zumindest teilweise ausgefüllt ist.

8. Distraktionsvorrichtung nach einem der Ansprüche 1 bis 5,
**dadurch gekennzeichnet,**
**dass** der Verstärkungseinsatz (25) das Innere einer rohrförmigen, an der Innenwand des Zylinders (17) geführten Kolbenstange (27) zumindest über einen Teilumfangsbereich vollständig ausfüllt.

9. Distraktionsvorrichtung nach einem der vorhergehenden Ansprüche,
**dadurch gekennzeichnet,**
**dass** die Kolben-/Zylinder-Anordnung (15, 17) im zusammengesetzten Zustand mit den Arbeitsabschnitten (11, 13) koppelbar ist.

10. Distraktionsvorrichtung nach einem der vorhergehenden Ansprüche,
**dadurch gekennzeichnet,**
**dass** wenigstens ein Verstärkungseinsatz (21, 25) fest mit dem einen Arbeitsabschnitt (11) verbunden ist.

11. Distraktionsvorrichtung nach einem der vorhergehenden Ansprüche,
**dadurch gekennzeichnet,**
**dass** wenigstens ein Verstärkungseinsatz (23) in Form eines separaten Bauteils vorgesehen ist.

12. Distraktionsvorrichtung nach einem der vorhergehenden Ansprüche,
**dadurch gekennzeichnet,**
**dass** die Stützaufnahme (19) für den Zylinder (17) und der mit dem Zylinder (17) koppelbare Arbeitsabschnitt (13) fest mit einem Tragkörper (43) verbunden sind.

13. Distraktionsvorrichtung nach einem der vorhergehenden Ansprüche,
**dadurch gekennzeichnet,**
**dass** auf der den Arbeitsabschnitten (11, 13) gegenüberliegenden Seite der Kolben-/Zylinder-Anordnung (15, 17) ein Handgriff (45) vorgesehen ist, der bevorzugt fest mit einem Tragkörper (43) verbunden ist.

14. Distraktionsvorrichtung nach einem der vorhergehenden Ansprüche,
**dadurch gekennzeichnet,**
**dass** der mit dem Zylinder (17) koppelbare Arbeitsabschnitt (13) fest mit einem sich parallel zur Distraktionsrichtung erstreckenden Funktionsblock (47) verbunden ist, der als Verdreh- und/oder Auslenksicherung für den mit dem Kolben (15) koppelbaren Arbeitsabschnitt (11) ausgebildet ist.

15. Distraktionsvorrichtung nach Anspruch 14,
**dadurch gekennzeichnet,**
**dass** der Funktionsblock (47) einen von einer Kreisform abweichenden Außenquerschnitt aufweist und zumindest über einen Teilumfangsbereich von einem Führungsabschnitt (49) des Arbeitsabschnitts (11) verdrehsichernd umgriffen ist.

16. Distraktionsvorrichtung nach Anspruch 14 oder 15,
**dadurch gekennzeichnet,**
**dass** der Funktionsblock (47) eine geringfügig gegenüber der Distraktionsrichtung geneigte, in Richtung zunehmender Distraktionslänge auf die Längsachse der Kolben-/Zylinder-Anordnung (15, 17) zu laufende Anschlagfläche (51) aufweist, die mit dem Arbeitsabschnitt (11) auslenkungssichernd zusammenwirkt.

17. Distraktionsvorrichtung nach einem der Ansprüche 14 bis 16,
**dadurch gekennzeichnet,**
**dass** der Funktionsblock (47) mit einer Anzeigeeinrichtung (53), insbesondere in Form einer an einer Außenseite angebrachten Skala, versehen ist, an der ein Maß für die Distraktionslänge ablesbar ist.

18. Distraktionsvorrichtung nach einem der vorhergehenden Ansprüche,
**dadurch gekennzeichnet,**
**dass** die Stützaufnahme (19) und/oder der Verstärkungseinsatz (21, 23, 25) aus einem Material mit einer höheren Biegesteifigkeit als der Kunststoff der Kolben-/Zylinder-Anordnung (15, 17) hergestellt sind, insbesondere aus Metall oder einem faserverstärkten Kunststoff.

19. Distraktionsvorrichtung nach einem der vorhergehenden Ansprüche,
**dadurch gekennzeichnet,**
**dass** die Kolben-/Zylinder-Anordnung (15, 17) mit einer Anzeigeeinrichtung, insbesondere in Form einer am Zylinder angebrachten Skala, versehen ist.

20. Distraktionsvorrichtung nach einem der vorhergehenden Ansprüche,
**dadurch gekennzeichnet,**
**dass** eine Fluidzuführeinrichtung (33) für die Kolben-/Zylinder-Anordnung (15, 17) eine von Hand betätigbare Spritze (35) umfasst, insbesondere eine Einwegspritze.

21. Distraktionsvorrichtung nach einem der vorhergehenden Ansprüche,
**dadurch gekennzeichnet,**
**dass** an eine die Kolben-/Zylinder-Anordnung (15, 17) mit einer Fluidzuführeinrichtung (33) verbindende Fluidleitung (37) wenigstens eine Druckanzeigeeinrichtung (39) angeschlossen ist.

22. Distraktionsvorrichtung nach einem der vorhergehenden Ansprüche,
**dadurch gekennzeichnet,**
**dass** die Kolben-/Zylinder-Anordnung (15, 17) ein Bestandteil eines geschlossenen, als eine Einheit handhabbaren, insbesondere sterilisierbaren, Hydrauliksystems ist, das zusätzlich zumindest eine Fluidzuführeinrichtung (33), eine die Kolben-/Zylinder-Anordnung (15, 17) mit der Fluidzuführeinrichtung (33) verbindende Fluidleitung (37) sowie ein Hydraulikfluid, insbesondere Wasser, umfasst und als Ganzes mit den Arbeitsabschnitten (11, 13) koppelbar ist.

23. Distraktionsvorrichtung nach einem der vorhergehenden Ansprüche,
**dadurch gekennzeichnet,**
**dass** wenigstens zwei im mit den Arbeitsabschnitten (11, 13) gekoppelten Zustand in parallelen Richtungen wirksame Kolben-/Zylinder-Anordnungen (15, 17) vorgesehen sind.

24. Distraktionsvorrichtung nach Anspruch 23,
**dadurch gekennzeichnet,**
**dass** die Kolben-/Zylinder-Anordnungen (15, 17) während der Benutzung an eine gemeinsame Fluidzuführeinrichtung (33) angeschlossen sind.

25. Distraktionsvorrichtung nach Anspruch 23 oder 24,
**dadurch gekennzeichnet,**
**dass** durch in einer die Kolben-/ Zylinder-Anordnungen (15, 17) mit einer Fluidzuführeinrichtung (33) verbindenden Fluidleitung (37) angeordnete Schaltventile (41) die Kolben-/Zylinder-Anordnungen (15, 17) unabhängig voneinander jeweils wahlweise mit der Fluidzufuhr verbindbar oder von der Fluidzufuhr trennbar sind.

26. Distraktionsvorrichtung nach einem der vorhergehenden Ansprüche,
**dadurch gekennzeichnet,**
**dass** zum Einstellen von Sollabständen zwischen Femur (61) und Tibia (63) die Arbeitsabschnitte (11, 13) einen zwischen Femur (61) und Tibia (63) einbringbaren und sich im Wesentlichen senkrecht zur Distraktionsrichtung erstreckenden Aufspreizabschnitt bilden.

27. Distraktionsvorrichtung nach einem der vorhergehenden Ansprüche,
**dadurch gekennzeichnet,**
**dass** zur Bestimmung der Bandspannung und/oder Bandlänge bei der Implantation von vorderen Kreuzbändern (65) ein oberer Arbeitsabschnitt (11) mit einem Abstütz- und/oder Befestigungsabschnitt (67, 81) versehen ist, über den die Vorrichtung an der Tibia (63) abstützbar und/oder befestigbar ist.

28. Distraktionsvorrichtung nach Anspruch 27,
**dadurch gekennzeichnet,**
**dass** die Arbeitsabschnitte (11, 13) jeweils mit wenigstens einer Aussparung (69) zum Hindurchführen eines Fixierwerkzeugs (71) und eines Fixierelementes zum Fixieren des Bandes (65) in der Tibia (63) versehen sind.

29. Distraktionsvorrichtung nach Anspruch 27 oder 28,
**dadurch gekennzeichnet,**
**dass** der obere Arbeitsabschnitt (11) mit wenigstens einer Aussparung (69) zum Hindurchführen des Bandes (65) und/oder eines mit dem Band (65) verbundenen Zugelementes (73) versehen ist.

30. Distraktionsvorrichtung nach einem der Ansprüche 27 bis 29,
**dadurch gekennzeichnet,**
**dass** ein unterer Arbeitsabschnitt (13) mit wenigstens einem Befestigungsabschnitt zum Befestigen des Bandes (65) oder eines mit dem Band (65) verbundenen Zugelementes (73) versehen ist.

31. Distraktionsvorrichtung nach einem der vorhergehenden Ansprüche,
**dadurch gekennzeichnet,**
**dass** die Arbeitsabschnitte (11, 13) jeweils als zungenförmige Plattformen ausgebildet sind, die im zusammengesetzten Zustand zumindest im Wesentlichen parallel zueinander verlaufen.
